# EUROPEAN PATENT APPLICATION

(11) **EP 1 038 965 A1**
(43) Date of publication of application: **27.09.2000**
(21) Application number: 99105073.3
(22) Date of filing: 23.03.1999
(51) Int. Cl.: C12N 15/55, C12N 9/16, C12Q 1/68, C07K 16/40, A01H 5/00, A01H 5/10

(54) **Method of screening for chemical compounds capable of inducing ERS in plants**

(71) Applicant: AMERICAN CYANAMID COMPANY, Madison, New Jersey 07940 (US)
(72) Inventor: Jarosch, Birgit Dipl.-Biol., 52072 Aachen (DE)
(74) Representative: Wibbelmann, Jobst, Dr., Dipl.-Chem.

(57) **Abstract**

The invention relates to a method of testing a chemical compound for its capability to induce an enhanced resistance status (ERS) in plants said method comprising:
(a) contacting a plant or plant part comprising a gene fragment coding for an acid phosphatase the expression of which being inducible by SAR inducing compounds, and in particular both by SAR inducing compounds and ISR inducing compounds, with the chemical compound; and
(b) assaying the expression of said gene fragment, wherein the expression of said gene fragment indicates that the chemical compound has the capability to induce ERS. In a particular embodiment the gene fragment codes for an acid phosphatase from *Hordeum vulgare.* Further aspects of the invention are DNA molecules comprising said particular gene fragment the expression of which may be assayed in the method according to the invention, transgenic plants, plant parts or seeds comprising said DNA molecules, polypeptides or proteins encoded by said DNA molecules and antibodies specifically recognizing and binding said polypeptides or proteins.

## Description

### BACKGROUND OF THE INVENTION

This invention concerns a method of testing a chemical compound for its capability to induce an enhanced resistance status (ERS) against pathogens in plants, and to the use of said method for screening for chemical compounds having the capability to induce ERS in plants. Under further aspects, the invention pertains to DNA molecules encoding a polypeptide having the biological activity of the enzyme acid phosphatase the expression of which, within their natural genetic environment, may be induced by ERS inducing compounds and may be assayed in the method according to the invention, transgenic plants, plant parts or seeds comprising said DNA molecules, polypeptides or proteins encoded by said DNA molecules and antibodies specifically recognizing and binding said polypeptides or proteins.

An enhanced resistance status (ERS) in plants is the result of biologically and/or chemically induced resistance phenomena which inter alia include systemic acquired resistance (SAR) and induced systemic resistance (ISR) mediated by Rhizobacteria.

In the last decade convincing data have been accumulated that salicylic acid (SA) is essential for the establishment of systemic acquired resistance (SAR) of dicotyledonous plants against a broad range of pathogens (e.g. Yang et al., Genes and Development 11, 1621-1639 (1997)) thus leading to an enhanced resistance status (ERS) in those plants.

In contrast to SAR, other plant defence reactions leading to ERS which are summarized under the term ISR, such as resistance mediated by Rhizobacteria (Ann. Rev. Phytopathology 36, 453-483 (1998)) as well as the jasmonate-mediated wound response including expression of the proteinase inhibitor 1 and 2 gene, are not inducible by salicylic acid or other SAR inducing compounds and are sometimes even inhibited by salicylic acid or derivatives thereof (e.g. Doares et al., Plant Physiol. 108, 1741-1746 (1995)).

The US Patent 5,614,395 discloses a method for screening for SAR inducing agrochemicals with the aid of genetically modified plants containing a chimeric gene which responds on SAR inducing compounds, which is disclosed as a wheat gene such as *WCI-1.* This method is limited to those plant species which are susceptible for the chimeric gene, in particular to wheat.

The International Patent application WO 98/00023 suggests a method of screening compounds for resistance-inducing activity by the detection of the expression of a plant defensin gene such as *PDF 1.* The genes disclosed there are induced by plant pathogens such as *Alternaria brassicicola* or *Botrytis cinera* but not by SAR inducing agrochemicals such as salicylic acid or dichloroisonicotinic acid.

Besides their enzymatic activity, acid phosphatases appear to play a major role as vegetative storage proteins within plants, i.e. as an amino acid reservoir. In soybean two genes (vspA and vspB) code for corresponding vegetative storage proteins - acid phosphatases - which are located in the vacuole. These proteins accumulate to very high levels in hypocotyls, young leaves, flowers and pods but transcripts are rare in mature stems, leaves and roots. Gene expression is modulated by water deficit, wounding, sugars, light, jasmonic acid and methyl jasmonate. It is suggested that inhibition of growth necessitates temporary storage of amino acids (Mason et al., 1990, 1993; Berger et al., 1995).

However, up to now responsiveness on SAR inducing compounds has not been reported for genes encoding acid phosphatase enzymes. Accordingly, there has been no motivation to use naturally occurring acid phosphatase genes for screening ERS inducing compounds including SAR inducing compounds.

The generation of synthetic combinatorial libraries is well known for example from EP 0 734 530, EP 0 816 309 and EP 0 818 431. However, up to now it was only possible to screen such libraries in order to select compounds with pharmaceutical properties. Therefore, it was highly desirable to develop a high through-put screening method which allows to generate lead compounds for the agricultural research, in particular for ERS inducing compounds.

### SUMMARY OF THE INVENTION

The present invention is based on the most surprising finding that there exist genes encoding acid phosphatase enzymes the expression of which is inducible by SAR inducing compounds, and in a particular embodiment by both SAR inducing compounds and ISR inducing compounds.

This finding enables the establishment of novel methods for testing a chemical compound for its capability to induce an enhanced resistance status (ERS), and particularly systemically acquired resistance (SAR) or induced systemic resistance (ISR), in plants and for screening a multiplicity of chemical compounds, in particular agrochemicals, for compounds or compositions having the capability to induce ERS in plants.

The present invention relates to a method of testing a chemical compound for its capability to induce an enhanced resistance status (ERS) in plants said method comprising:
(a) contacting a plant or plant part comprising a gene fragment coding for an acid phosphatase the expression of which being inducible by SAR inducing compounds, with the chemical compound; and
(b) assaying the expression of said gene fragment, wherein the expression of said gene fragment indicates that the chemical compound has the capability to induce ERS.

In a particular embodiment, the expression of the said gene fragment coding for an acid phosphatase the expression of which being inducible by SAR inducing compounds is not inducible by plant pathogens. In a further preferred embodiment, the expression of the said gene fragment is additionally inducible also by ISR inducing compounds.

Furthermore, this method according to the present invention allows to screen ERS inducing compounds in different plant species as for example cereals such as wheat or barley, and rice; to determine the presence of ERS-inducing compounds in a sample; to determine the concentration of an ERS-inducing compound in a sample; to compare two ERS-inducing compounds for their relative capabilities to induce ERS in plants; and to test compounds and compositions for their capability to change the ERS inducing effect of a chemical compound.

A further aspect of the present invention relates to a kit for use in the methods of the present invention.

Another aspect of the present invention relates to a process for providing compounds having the capability of inducing ERS in plants which comprises the steps of
(a) generating a synthetic combinatorial library of different chemical compounds, and
(b) screening the compounds of said library with the screening method according to this invention. Further aspects of the invention are the novel compounds which are obtainable with the aid of this method and the use thereof as agrochemicals.

Under further aspects, the invention pertains to DNA molecules encoding a polypeptide having the biological activity of the enzyme acid phosphatase the expression of which may be assayed in the methods according to the invention, fragments of said DNA molecules which i.a. might be used as probes or primers, transformed microorganisms and transgenic plants, plant parts or seeds comprising said DNA molecules, polypeptides and proteins encoded by said DNA molecules and antibodies specifically recognizing and binding said polypeptides or proteins.

These and other objects and features of the invention will become apparent from the detailed description set forth hereinbelow.

### BRIEF DESCRIPTION OF THE DRAWINGS

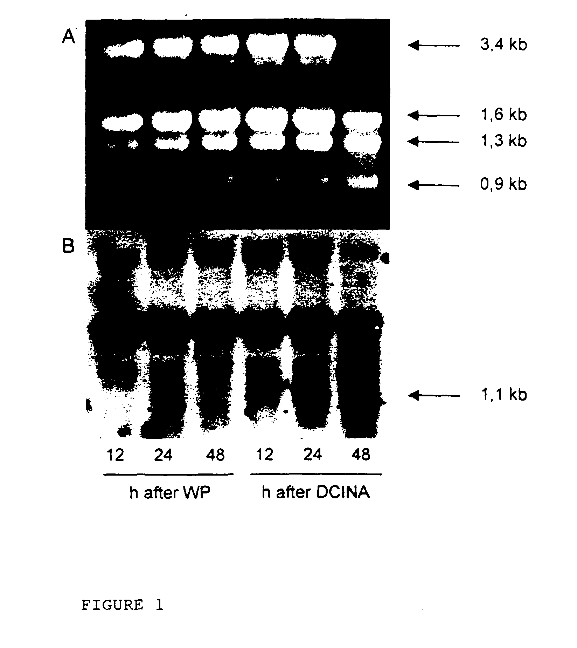

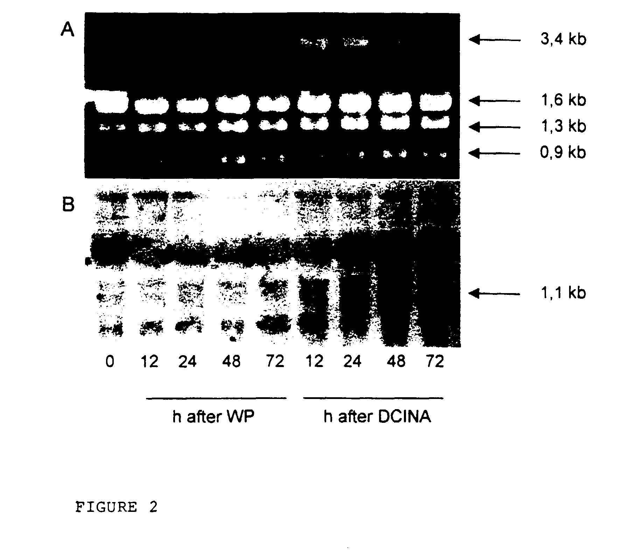

FIGS. 1 and 2: Time course experiments of 2,6-Dichloroisonicotinic acid (DCINA)-induced expression of the acid phosphatase gene from *Hordeum vulgare L.* Northern (RNA) analysis of the acid phosphatase gene after treatment of barley plants (mutant A89) with DCINA and the wettable powder carrier (WP) alone, respectively. In Figures 1 and 2, the results of each of duplicate experiments are shown, respectively. Tissue was harvested at the time points indicated. 10 µg of total RNA was loaded per lane. Equal loading was checked by fluorescence of rRNA with ethidiumbromide under UV light (A). Blots were hybridized with Dig-labeled PCR fragments at 68°C and washed under stringent conditions two times with 2 x SSC/0,1% SDS at room temperature and three times with 0,1 x SSC/0,1% SDS at 68°C (B). Equally, the phosphatase gene expression is induced by the compounds Bion®, a product sold by Novartis, and jasmonic acid (figures not shown).

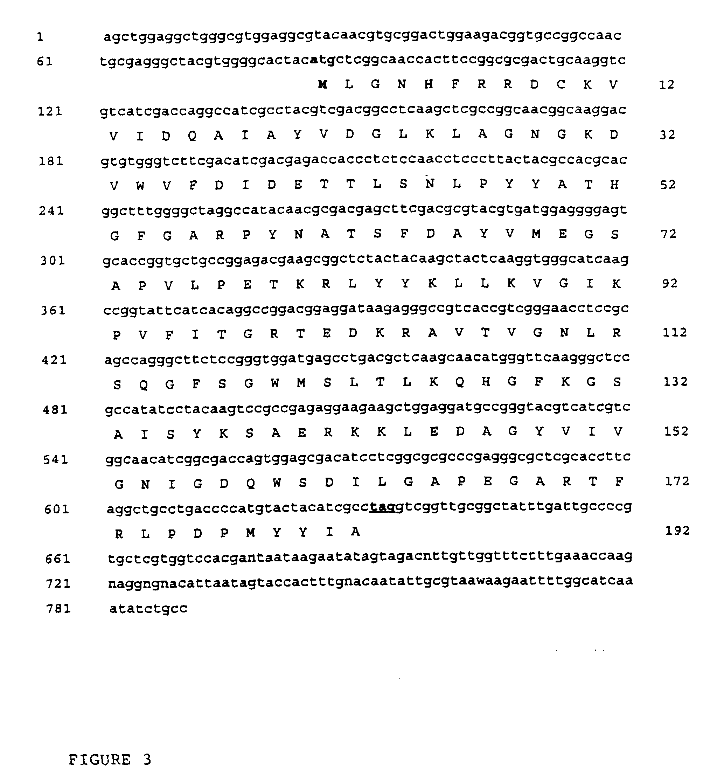

FIG. 3: Nucleotide sequence of acid phosphatase cDNA from *Hordeum vulgare L.* and deduced amino acid sequence. The numbers on the left margin refer to nucleotides; the numbers on the right margin refer to amino acid residues.

### DETAILED DESCRIPTION OF THE PREFERRED EMBODIMENTS

It has now been found that the capability of chemical compounds to induce ERS in plants can be efficiently tested by
(a) contacting a plant or plant part comprising a gene fragment coding for an acid phosphatase the expression of which being inducible by SAR inducing compounds, and in particular both by SAR inducing compounds and ISR inducing compounds, with the chemical compound; and
(b) assaying the expression of said gene fragment.

By use of said method, a multiplicity of chemical compounds and compositions may be screened to identify compounds and/or compositions having the capability to induce ERS in plants. In a particular embodiment said multiplicity of chemical compounds or compositions may be provided, for example, in form of a combinatorial library such as exemplified in EP 0 734 530, EP 0 816 309 and EP 0 818 431, or in form of a library or collection of natural compounds and/or compositions or a library comprising both natural and synthetic compounds and compositions.

Alternatively, the said method may also be used for determining the presence of compounds capable of inducing ERS in plants in a sample, wherein in the above step (a) the plant or plant part is contacted with an aliquot of the sample in place of the chemical compound. The expression of the said gene fragment then indicates the presence of one or more compounds capable of inducing ERS in plants in the sample.

In a particularly preferred embodiment the expression of the said gene fragment in the said plant which is inducible by SAR inducing compounds, and preferably both by SAR inducing compounds and ISR inducing compounds, is not inducible by naturally occurring plant pathogens. This feature appears to be particularly valuable if it is intended to perform the methods of the present invention in an environment where plant pathogens may be present. This will be the case when using plants in a green house or on the field for performing the methods of the present invention. In these cases, said specific feature will ensure that any expression of the said gene fragment observed will only be attributable to the presence of a compound or composition having the capability to induce ERS in plants. If working the method according to the invention in a cell or tissue culture system, however, said specific feature may be utilized but it is not essential.

In the present context, the phrase "naturally occurring plant pathogens" includes all kinds of phytopathogenic microorganisms, in particular phytopathogenic fungi, bacteria and viruses.

The phrases "plants" and "plant parts" as used hereinabove and hereinbelow include all kinds of plants and plant parts, in particular foliage, roots and seeds. Preferably the said plant or plant part is selected from the group consisting of a plant protoplast, a plant cell, a plant tissue, callus, a developing plantlet, a plant leaf, an immature whole plant, a mature whole plant and seed. In particular, the said plant or plant part, in particular plant cell or plant leaf, is or is derived from a monocotyledonous plant, such as a cereal, particularly wheat or a plant of the genus Hordeum such latter plants being particularly preferred, rice as well as from dicotyledonous plants.

The gene fragment of step (a) may be any gene fragment encoding an acid phosphatase enzyme the expression of which being inducible by SAR inducing compounds, and preferably both by SAR inducing compounds and ISR inducing compounds. This definition comprises any naturally occurring gene fragments exhibiting these characteristics as well as not-naturally occurring gene fragments, the sequence of which has substantial homology with the sequence of any of said naturally occuring gene fragments, variants thereof as well as gene fragments comprising one or more portions of the aforementioned gene fragments and variants wherein all these gene fragments and variants maintain the characteristic indicated above, i.e. an expression which is inducible by SAR inducing compounds, and preferably both by SAR inducing compounds and ISR inducing compounds.

As already mentioned above, in a particular embodiment of the invention, the expression of the said gene fragment or variant which is inducible by SAR inducing compounds, and preferably both by SAR inducing compounds and ISR inducing compounds, is not inducible by naturally occurring plant pathogens.

In a preferred embodiment of the present invention, the gene fragment of step (a) corresponds to a gene fragment consisting of, essentially consisting of or comprising the following nucleotide sequence:

ATGCTCGGCAACCACTTCCGGCGCGACTGCAAGGTCGTCATCGACCAGGCCATC
GCCTACGTCGACGGCCTCAAGCTCGCCGGCAACGGCAAGGACGTGTGGGTCTTC
GACATCGACGAGACCACCCTCTCCAACCTCCCTTACTACGCCACGCACGGCTTT
GGGGCTAGGCCATACAACGCGACGAGCTTCGACGCGTACGTGATGGAGGGGAGT
GCACCGGTGCTGCCGGAGACGAAGCGGCTCTACTACAAGCTACTCAAGGTGGGC
ATCAAGCCGGTATTCATCACAGGCCGGACGGAGGATAAGAGGGCCGTCACCGTC

GGGAACCTCCGCAGCCAGGGCTTCTCCGGGTGGATGAGCCTGACGCTCAAGCAA
CATGGGTTCAAGGGCTCCGCCATATCCTACAAGTCCGCCGAGAGGAAGAAGCTG
GAGGATGCCGGGTACGTCATCGTCGGCAACATCGGCGACCAGTGGAGCGACATC
CTCGGCGCGCCCGAGGGCGCTCGCACCTTCAGGCTGCCTGACCCCATGTACTAC
ATCGCCTAG.

This sequence corresponds to the coding region plus the stop codon of the cDNA of an acid phosphatase from *Hordeum vulgare L.* and represents the region from nucleotide 85 to nucleotide 633 of the sequence given in SEQ. ID. NO. 1 of the attached sequence protocol. It is to be noted that the expression of this gene fragment is inducible both by SAR inducing compounds and ISR inducing compounds and is not inducible by naturally occurring plant pathogens.

In an alternative embodiment, the said gene fragment is a gene fragment, the nucleotide sequence of which exhibits substantial homology with the nucleotide sequence given above, or a variant thereof or comprises one or more portions of the nucleotide sequence given above, of a sequence having substantial homology thereto or a variant thereof. Accordingly, the term also includes sequences that can substantially hybridize to the nucleotide sequence given above under stringent conditions, in particular under conditions of low stringency. It also includes DNA which hybridizes to the gene fragment having the nucleotide sequence depicted in SEQ. ID. NO. 1 or a part of it and which codes for at least part of the gene sequence.

The phrase "variant thereof" as used hereinabove and hereinbelow means any substitution, variation, modification, insertion, deletion or addition of one or more nucleotides from or to the nucleotide sequence of a naturally occurring gene fragment coding for an acid phosphatase enzyme the expression of which being inducible by SAR inducing compounds, and preferably both by SAR inducing compounds and ISR inducing compounds, for example a gene fragment comprising the nucleotide sequence given above, provided that the expression of the variant being still inducible by application of SAR inducing compounds, and preferably both of SAR inducing compounds and ISR inducing compounds. The phrase particularly includes allelic variants of naturally occurring gene fragments coding for an acid phosphatase enzyme the expression of which being inducible by SAR inducing compounds, and preferably both by SAR inducing compounds and ISR inducing compounds, for example of the gene fragment comprising the nucleotide sequence given above or in SEQ. ID. NO. 1. The phrase also includes synthetic variants comprising or essentially consisting of nucleotide sequences that can substantially hybridize to the nucleotide sequence of the naturally occurring parent gene fragment. It also includes DNA which hybridizes to the nucleotide sequence of the parent gene fragment, and which codes for at least part of the acid phosphatase gene product of the said gene fragment. Preferably, such hybridisation occurs at low-stringency conditions, in a further embodiment also between low and high stringency conditions, and, in a very particular embodiment, even at high stringency conditions. As a rule low stringency conditions can be defined as 3 x SSC at ambient temperature to 65°C and high stringency conditions as 0.1 x SSC at 68°C. SSC is the abbreviation of a 0.15M sodium chloride, 0.015M trisodium citrate buffer.

The phrase "substantial homology" as used hereinabove and hereinbelow embraces homology with respect to at least the essential nucleotide/s of the parent gene sequence encoding an acid phosphatase, e.g., the gene sequence the nucleotide sequence of which is given in SEQ. ID. NO. 1, provided that the expression thereof still being inducible by SAR inducing compounds, and preferably both by SAR inducing compounds and ISR inducing compounds. Preferably, the homologous sequence exhibits an activity corresponding to the activity of the naturally occurring parent acid phosphatase gene fragment. Typically, homology is shown when 60 % or more of the nucleotides are common with the naturally occurring parent gene fragment, more typically 65 %, preferably 70 %, more preferably 75 %, even more preferably 80 or 85 % and particularly preferred are 90 %, 95 %, 98 % or 99 % or more homology.

In a particular embodiment of the present method, the gene fragment coding for an acid phosphatase the expression of which being inducible by SAR inducing compounds, and preferably both by SAR inducing compounds and ISR inducing compounds, further comprises a nucleotide sequence coding for an indicator protein or polypeptide fused to the acid phosphatase coding region. Consequently, also the expression of the indicator protein or polypeptide is inducible by SAR inducing compounds, and preferably both by SAR inducing compounds and ISR inducing compounds, and, upon application of ERS inducing compounds, occurs concomitantly with the expression of the acid phosphatase activity in form of a fusion protein with the protein or polypeptide having the acid phosphatase activity. In specific embodiments said nucleotide sequence coding for an indicator protein or polypeptide codes for a glucuronidase enzyme or for the green fluorescent protein (GFP).

The SAR inducing compounds and ISR inducing compounds which may be assayed with the methods according to the invention, may be summarized under the generic term "ERS inducing compounds". This term includes all compounds being capable to provide a plant with improved resistance characteristics against all types of pests, for example against harmful microorganisms, like phytopathogenic fungi, bacteria and viruses. As already stated, it includes i.a. all compounds being capable to induce systemic acquired resistance (SAR) and induced systemic resistance (ISR) including defense mechanisms independent of salicylic acid in plants. ERS inducing compounds induce in the plants a resistance status by activating, for example, cell death reactions (CHR), cell wall appositions, PR-gene expression and/or phytoalexin accumulation.

The application of these compounds leads to a protection of the treated plants from serious infection by harmful microorganisms, for example phytopathogenic fungi, bacteria and viruses.

SAR inducing chemical compounds include benzoic acid, salicylic acid, dichloroisonicotinic acid, polyacrylic acid, aminobutyric acid, arachidonic acid and derivatives thereof.

Another group of SAR inducing chemical compounds are benzo-1,2,3-thiadiazole derivatives as for example disclosed by US Patents US 4,931,581 and US 5,229,384.

The reference compound of the ISR inducing chemical compounds is represented by jasmonic acid and derivatives thereof such as methyl jasmonate.

The known ERS inducing compounds are preferably used as reference compounds for the screening of novel agrochemical compounds.

Other chemical compounds, in particular agrochemicals, encompassed by the present invention, in particular individual members of a synthetic combinatorial library, can be determined easily by assaying the test chemical with the aid of the methods according to this invention.

The ERS inducing chemical compounds may be applied in pure form, in solution or suspension, as powders or dusts, or in other conventional formulations used agriculturally. Such formulations may include solid or liquid carriers, that is, materials with which said ERS inducing chemical compound is combined to facilitate application to the plant, tissue, cell or tissue culture, or to improve storage, handling or transport properties. Examples of suitable carriers include silicates, clays, resins, alcohols, ketones, aliphatic or aromatic hydrocarbons, and the like. If formulated as conventional wettable powder, aqueous suspension or emulsion concentrate, the formulation of the ERS inducing chemical compound may include one or more conventional surfactants, either ionic or non-ionic, such as wetting agents, emulsifying or dispersing agents.

The ERS inducing chemical compounds may also be applied in combination with another agent, for example with an adjuvant, a herbicide, a fungicide, an insecticide, a growth regulator or a fertilizer.

As a liquid formulation the ERS inducing chemical compound may be applied as a spray to plant leaves, stems or branches or to seeds before planting or to soil or other growing or cultivation medium.

The ERS inducing chemical compound is applied in an amount and over a period of time sufficient to induce ERS effects. If applied to whole plants, as a rule, the chemicals are applied in a concentration of 0.1 to 1000 mg active ingredient per liter of soil volume in a soil drench process or, alternatively, as a spray in a concentration of 0.1 - 100 mg active ingredient per liter of spray solution. If working in a cell or tissue culture system, also lower concentrations, now based on the volume of the culture medium, may be used. As a guideline 6 to 72 hours, in particular 12 to 48 hours are sufficient to detect a response.

In principle every conventional method of assaying the expression of genes can be used to monitor the response of the plant on the action of the chemical compound, such as Western blot or Northern blot analysis, detection or monitoring of the expression of an indicator protein or polypeptide the gene thereof being included in said gene fragment the expression of which being inducible by a SAR inducing compound, e.g. by detection of an enzymatic activity thereof. If the expression which has to be detected occurs tissue-specific, the analysis will be performed on said specific tissue.

If there exists a constitutive low level expression of the said gene fragment or of a part of the said gene fragment which will enable the detection of the expression thereof in the plant or plant part in the absence of the chemical compound, the expression induced by the chemical compound will be determined as an increase in expression compared to said constitutive low level expression. This assaying variant is also encompassed by step (b) of the method according to the invention.

Preferably, the analysis of the expression of the said gene fragment is carried out using a Northern blot type analysis, in particular with digoxigenin labelled cDNAs of the gene fragment, e.g. a cDNA with a nucleotide sequence as given in SEQ. ID. NO. 1, or fragments thereof, most preferred using a commercially available Northern blot kit such as the DIG-High Prime labelling Kit of Boehringer Mannheim, Germany.

In another preferred embodiment of the present invention the analysis of the expression of the said gene fragment is carried out using antibodies to detect the or a gene product of the said gene fragment, e.g. the protein or polypeptide having acid phosphatase activity or a specific indicator polypeptide or protein the gene sequence of which representing a part of the said gene fragment, e.g. by performing a Western blot type analysis.

If the detection of the expression of the said gene fragment is based on the detection of the expression of an "additional" nucleotide sequence comprised within the said gene fragment and encoding an indicator protein or polypeptide, the detection may be performed also by other means than by use of nucleotide probes (e.g. for a Northern-blotting assay) or antibodies (e.g. for a Western-blotting assay), depending on the type of indicator protein or polypeptide produced. The indicator protein or polypeptide may be a fluorescent or chemiluminescent protein or polypeptide, such as the green fluorescent protein (GFP), thus enabling the detection of expression of the said gene fragment by detection of fluorescence or chemiluminescence of the said plant cells. Alternatively, the indicator protein or polypeptide may also exhibit an enzymatic activity not naturally occurring in the plant or plant part used for the method of the invention. Accordingly, this enzymatic activity may be assayed for detection of the expression of the indicator protein or polypeptide and, accordingly, of the said gene fragment. The assay may be based on the measuring of the disappearance of a substrate for the enzyme from an assay medium or on the measuring of the formation of a product by the enzymatic conversion. In a particular embodiment, said product of the enzymatic conversion may be a compound which, as a result of the enzymatic conversion, is coloured. An example for an indicator protein with an enzymatic activity, which usually will not be found in plants, is the glucuronidase enzyme.

The assaying step (b) for assaying the expression of the said gene fragment may be carried out in a way
- to produce a result which enables the mere determination whether or not an expression has occurred;
- to enable the determination which of two or more samples shows a higher degree of expression of the said gene fragment compared to the other(s) ("semi-quantitative determination");
- to provide quantitative results as to the level of expression occurred ("quantatitve determination").

If care is taken to perform the assaying step (b) with identical or substantially identical amounts of nucleotide or protein material prepared from the step (a) treated plants or plant parts, thus enabling a semi-quantitative determination or even quantitative determination of the level of expression of the said gene fragment, the method according to the invention may also be used
-- for determining the concentration of a chemical compound which is capable of inducing ERS in plants in a sample;
-- for determining whether a chemical compound or composition exhibits a stronger ERS inducing effect than an ERS inducing reference compound; and
-- for determining whether and, optionally, to what degree a compound or composition is capable of changing the ERS inducing effect of a chemical compound.

The method for determining the concentration of a chemical compound which is capable of inducing ERS in plants in a sample, is characterized in that it comprises the following steps:
(i) performing the method according to the invention comprising steps (a) and (b) as outlined above wherein in step (a) the plant or plant part is contacted with an aliquot of the sample;
(ii) determining the concentration of the ERS inducing chemical compound in the sample by comparing the result from step (i) with the results of corresponding tests performed with known concentrations of said chemical compound.

The method for determining whether a chemical compound exhibits a stronger ERS inducing effect than an ERS inducing reference compound, such as salicylate, dichloroisonicotinic acid or jasmonic acid, is characterized in that it comprises the following steps:
(i) performing the method according to the invention comprising steps (a) and (b) as outlined above wherein in step (a) the plant or plant part is contacted with a specific concentration of the chemical compound;
(ii) performing the method according to the invention comprising steps (a) and (b) as outlined above wherein in step (a) the plant or plant part is contacted with an amount of the ERS inducing reference compound resulting in an equimolar concentration compared to the concentration of the chemical compound in step (i);
(iii) determining the relative level of the ERS inducing effect of the chemical compound by comparing the results from steps (i) and (ii).

Since any step (b) will be carried out using substantially equal amounts of nucleotide or protein material prepared from step (a) treated plant or plant part material for the assay, step (iii) will produce a semi-quantitative result in order to enable the determination which one of the chemical compound and the ERS inducing reference compound induces a higher expression of the said gene fragment and correspondingly will produce a stronger induction of ERS in a plant. Alternatively, the results may also be evaluated to enable a quantitative assessment of the relative degree of expression induced by the chemical compound compared to the ERS inducing reference compound. This evaluation may be performed for example by scanning of the analytical gels or films, photographs or print-outs thereof by means of a densitometer or by analyzing relevant excised sections of analytical gels used for separating assay mixtures comprising radioactively labelled detection compounds (e.g. nucleotide probes or antibodies) in a liquid scintillation counter.

The method for determining whether and, optionally, to what degree a compound or composition is capable of changing the ERS inducing effect of an agrochemical is characterized in that it comprises the following steps:
(i) performing the method according to the invention comprising steps (a) and (b) as outlined above wherein in step (a) the plant or plant part is contacted with a specific concentration of the agrochemical;
(ii) performing the method according to the invention comprising steps (a) and (b) as outlined above wherein in step (a) the plant or plant part is contacted with the same specific concentration of the agrochemical as in step (i) in the presence of the compound or composition;
(iii) determining the capability of the compound or composition for changing the ERS inducing effect of the agrochemical by comparing the results from steps (i) and (ii).
Since also in this method any step (b) will be carried out using substantially equal amounts of nucleotide or protein material prepared from step (a) treated plant or plant part material for the assay, step (iii) will produce a semi-quantitative result in order to enable the determination whether the compound or composition leads to an enhancement, reduction or even inhibition of the expression of the said gene fragment and correspondingly will produce a stronger or reduced induction of ERS in a plant or will even abolish the induction of ERS. Alternatively, the results may be evaluated to enable a quantitative assessment of the relative degree of expression induced by a combination of the agrochemical and the compound or composition compared to the agrochemical alone. This evaluation may be performed for example by scanning of the analytical gels or films, photographs or print-outs thereof by means of a densitometer or by analyzing relevant excised sections of analytical gels used for separating assay mixtures comprising radioactively labelled detection compounds (e.g. nucleotide probes or antibodies) in a liquid scintillation counter.

The method for determining whether and, optionally, to what degree a compound or composition is capable of changing the ERS inducing effect of an agrochemical may further be utilized in a method for screening for compounds or compositions capable of changing the ERS inducing effect of an agrochemical.

The invention further pertains to a kit for use in the methods according to the present invention. Said kits are characterized in that they comprise the following components:
(a) one or more plants or plant parts comprising a gene fragment coding for an acid phosphatase the expression of which being inducible by SAR inducing compounds, and preferably both by SAR inducing compounds and ISR inducing compounds; and
(b) means for detecting the expression of the said gene fragment.

In a particular embodiment the kit may comprise one or more plants or plant parts wherein the expression of the said gene fragment comprised therein which codes for an acid phosphatase is not inducible by plant pathogens. In a further particular embodiment the kit may comprise one or more plants or plant parts wherein the said gene fragment coding for an acid phosphatase additionally comprises a nucleotide sequence encoding an indicator protein or polypeptide.

If the kit comprises one or more plant parts, such as plant cells, said plant parts may be provided immobilized on a solid support. Suitable supports may i.a. be made of glass, specific polysaccharides, like agarose, or specific plastic materials such as polyacrylamide, polystyrene, polyvinylalcohol, silicones. Methods and techniques for immobilizing cells and tissue on such types of supports are within the skills of the skilled scientist. In a specific embodiment, in particular for the screening processes according to the present invention, the kit may comprise plates, such as microtiter plates, comprising a multiplicity of reaction wells which contain the said plant parts, in particular plant cells, preferably immobilized on the bottom and/or the walls of the wells.

The means for detecting the expression of the said gene fragment may comprise one or more nucleotide probe(s) such as detectably labelled cDNAs or cDNA fragments of the said gene fragment. The detectable label may be e.g. a fluorescent, chemiluminescent or radioactive label, and is in particular a digoxigenin molecule. Alternatively, for use in Western Blot type analyses, the means for detecting the expression of the said gene fragment will comprise an antibody which specifically recognizes and binds to the or a gene product of the said gene fragment. This antibody will carry a detectable label, e.g. a fluorescent or chemiluminescent dye, a radioactive label or an enzymatically active moiety or structure or will be detectable by use of a secondary antibody specific for said first antibody which may also be provided, separately from said first antibody, in the kit. For assaying an enzymatic activity of an indicator protein or polypeptide in the case where the said gene fragment the expression of which being inducible by ERS inducing compounds further comprises a nucleotide sequence coding for a respective indicator protein or polypeptide, the means for detecting the expression of the said gene fragment will comprise one or more reagents for assaying the enzymatic activity of the indicator protein or polypeptide including e.g. a substrate for the indicator protein, optionally in combination with one or more necessary cofactor(s), and possibly reagents for detecting the product from the enzymatic conversion of the substrate.

Furthermore, said means may comprise one or more buffers for the assay reaction(s) and/or reagents for determining and, in particular, visualization of the assay result (e.g. in the case of detection of the enzymatic activity attached to the antibody), which may be provided separately from each other and from the probes or antibodies or in combined form or in form of combinations of some of said components.

Optionally, the kit may comprise further components, in particular as separate components, such as one or more buffers for incubating the test compound or composition with a plant part, agents for breaking up the cellular structure of the plant or plant part for enabling the reactions for detection of the expression of the said gene fragment or instructions for use of the kit.

Furthermore, the invention pertains to a process for providing chemical compounds having the capability of inducing ERS in plants which comprises the steps of
(a) generating a synthetic combinatorial library of chemical compounds, and
(b) screening the compounds of said library with the method of testing a chemical compound for its capability to induce ERS in plants according to the invention comprising steps (a) and (b) as outlined above. The invention also covers the new compounds having the capability of inducing ERS which are obtainable by this method and the use thereof as agrochemical compounds, and in particular for the induction of ERS in plants. With respect to the formulation and application of the new agrochemical compounds for use as an agrochemical, reference is made to the relevant explanations given earlier in this specification.

Under a further aspect, the invention is directed to an isolated DNA molecule which codes for a protein or polypeptide having the biological activity of the enzyme acid phosphatase, the expression of which being inducible by SAR inducing compounds, and preferably both by SAR inducing compounds and ISR inducing compounds, if said DNA molecule is situated within its natural genetic environment, or a nucleotide sequence complementary thereto.

In a particular embodiment of the present invention the isolated DNA molecule comprises the following nucleotide sequence: 
ATGCTCGGCAACCACTTCCGGCGCGACTGCAAGGTCGTCATCGACCAGGCCATC
GCCTACGTCGACGGCCTCAAGCTCGCCGGCAACGGCAAGGACGTGTGGGTCTTC
GACATCGACGAGACCACCCTCTCCAACCTCCCTTACTACGCCACGCACGGCTTT
GGGGCTAGGCCATACAACGCGACGAGCTTCGACGCGTACGTGATGGAGGGGAGT
GCACCGGTGCTGCCGGAGACGAAGCGGCTCTACTACAAGCTACTCAAGGTGGGC
ATCAAGCCGGTATTCATCACAGGCCGGACGGAGGATAAGAGGGCCGTCACCGTC
GGGAACCTCCGCAGCCAGGGCTTCTCCGGGTGGATGAGCCTGACGCTCAAGCAA
CATGGGTTCAAGGGCTCCGCCATATCCTACAAGTCCGCCGAGAGGAAGAAGCTG
 
GAGGATGCCGGGTACGTCATCGTCGGCAACATCGGCGACCAGTGGAGCGACATC
CTCGGCGCGCCCGAGGGCGCTCGCACCTTCAGGCTGCCTGACCCCATGTACTAC
ATCGCCTAG,
 or a nucleotide sequence complementary thereto.

The expression of these DNA molecules may be assayed in the method according to the invention.

The invention further pertains to isolated DNA molecules the nucleotide sequence of which having substantial homology with the nucleotide sequence as given above, in particular hybridizes with the latter, or is a variant of the DNA molecule with the nucleotide sequence as given above or is related to said DNA molecule by mutation. With respect to the meaning of the terms "substantial homology" and "variant" reference is made to the explanations given earlier in this specification. In this context, specific embodiments are allelic variants to the DNA molecule having the nucleotide sequence given above or having the nucleotide sequence given in SEQ. ID. NO. 1 as well as DNA molecules having a nucleotide sequence which hybridizes to the nucleotide sequence given above or the nucleotide sequence as given in SEQ. ID. NO. 1. As already outlined above, preferably, such hybridization occurs at low stringency conditions, in a further embodiment between low and high stringency conditions, and in a very particular embodiment even at high stringency conditions. As a rule low stringency conditions can be defined as 3 x SSC at ambient temperature to 65°C and high stringency conditions as 0.1 x SSC at 68°C, SSC being the abbreviation of a 0.15M sodium chloride, 0.015M trisodium citrate buffer. A further specific embodiment are DNA molecules having a nucleotide sequence which not necessarily hybridizes with one of the nucleotide sequences given above and in SEQ. ID. NO. 1, respectively, but, due to the degeneration of the genetic code, encodes the identical amino acid sequence as given in SEQ. ID. NOS. 1 and 2.

In a specific embodiment a DNA molecule encoding a protein or polypeptide having the biological activity of the enzyme acid phosphatase the expression of which, in its natural genetic environment within a cell, being inducible by SAR inducing compounds, and preferably both by SAR inducing compounds and ISR inducing compounds, is derived from the genome of an acid phosphatase producing plant and may be identified and isolated by use of nucleotide hybridization probes consisting of or comprising fragments of the DNA molecule having the above given nucleotide sequence or the nucleotide sequence given in SEQ. ID. NO. 1. Preferred fragments of the said DNA molecules for the screening of acid phosphatase genes in other plants than *Hordeum vulgare L.,* and in particular in other plants of the genus *Hordeum,* further monocotyledonous and also dicotyledonous plants comprise at least 15 nucleotides, typically between 15 and 30 nucleotides, and more preferably at least 25 nucleotides, but may comprise, if necessary, even a higher number of nucleotides such as 50 or more nucleotides.

Accordingly, fragments of the DNA molecules of the present invention, and in particular of the isolated DNA molecule having the above given nucleotide sequence are also covered by the present invention as well as the use thereof as hybridization probes, particularly screening probes, or primers, in particular sequencing or PCR primers. The invention also covers fragments which are shorter than mentioned above, e.g. comprising between 8 and 15 nucleotides, in particular if they have utility as nucleotide probes or primers. Especially for use as nucleotide probes, DNA molecules comprising or consisting of one or more of the fragments according to the present invention are employed in labeled form, e.g. radioactively labeled or labeled by attachment of non-radioactive reporter or indicator molecules, such as digoxigenin molecule(s). Particularly for use as PCR primers, DNA molecules comprising or essentially consisting of one or more of the fragments according to the present invention are employed in a form which comprises modified nucleotide sequences within the sequence(s) of the inventive fragment(s) which provide for appropriate restriction sites within said primers and any amplified products after the PCR which facilitate cloning of said amplified products. Despite these nucleotide sequence modifications the PCR primers, under the PCR conditions, will still hybridize specifically with the target DNA molecule according to the present invention.

DNA fragments according to the present invention may be prepared, for example, on the basis of the nucleotide sequences given in the attached Sequence Protocol, possibly by use of appropriate restriction nucleases, or by chemical synthesis. Various suitable techniques for preparing DNA fragments and DNA molecules comprising one or more of these fragments according to the present invention are known to the skilled scientist.

Having identified an acid phosphatase gene in a different plant species by use of the nucleotide fragments or DNA molecules according to the invention as screening probes, it may be interesting to determine whether the expression thereof being inducible by ERS inducing compounds, in particular SAR inducing compounds, and preferably both by SAR inducing compounds and ISR inducing compounds. This may be performed, e.g., as outlined in the Examples, by cultivating the plant or plant parts in the presence of an ERS inducing compound, and analyzing said plant or plant parts then, for example, by Northern-blot analysis by use of hybridization probes specific for said acid phosphatase gene, e.g. the nucleotide fragments used for identification of said gene, comparing the results with the results from a cultivation of said plant or plant parts without the ERS inducing compound. Plants identified by the described method to comprise an acid phosphatase gene being inducible by ERS inducing compounds, in particular SAR inducing compounds, and preferably both by SAR inducing compounds and ISR inducing compounds, may then be used in the methods according to the present invention.

In a further particular embodiment the invention pertains to an isolated DNA molecule which comprises a DNA molecule or fragment as defined above fused with a nucleotide sequence coding for an indicator protein or polypeptide. In a preferred embodiment the complete encoded information of the said DNA molecule will be expressed if expressibly linked to a single expression control sequence. The indicator protein or polypeptide encoded by this further nucleotide sequence portion of the DNA molecule is a protein or polypeptide which is easily detectable, e.g. by use of specific nucleotide hybridization probes, by use of specific antibodies, or due to specific characteristics of the protein or polypeptide itself like fluorescence or enzymatic activity. In specific embodiments said further nucleotide sequence portion of the DNA molecule may code for glucuronidase or the green fluorescent protein. The isolation and cloning of appropriate genes for indicator polypeptides or proteins as well as the production of the recombinant chimeric DNA molecules defined above are well within the skills of a skilled scientist.

The invention also covers DNA constructs which comprise one or more of the DNA molecules and/or fragments defined above, and in particular DNA constructs serving as nucleotide probes or PCR primers, as well as constructs comprising the DNA molecule(s) and/or fragment(s) under the control of an expression control sequence, i.e. an expression control sequence operatively linked to said DNA molecule(s) and/or fragment(s).

Especially for use as nucleotide probes, DNA molecules comprising or consisting of one or more of the DNA molecules and fragments according to the present invention will be employed in a labeled form, wherein said labelling may be achieved by attachment of non-radioactive reporter or indicator molecules such as fluorescent or phosphorescent molecules, digoxigenin molecule(s), biotin molecule(s) or derivatives thereof.

Especially for use as PCR primers, DNA constructs comprising or essentially consisting of one or more of the DNA molecules and fragments according to the present invention are employed in a form which comprises additional nucleotide sequences or modified nucleotide sequences within the sequence(s) of the inventive fragment(s) which provide for appropriate restriction sites within said primers and any amplified products after the PCR which facilitate cloning of said amplified products. Despite these nucleotide sequence modifications the PCR primers, under the PCR conditions, will still hybridize specifically with the target DNA molecule according to the present invention. In a further particular embodiment the DNA construct further comprises an expression control sequence which is operatively linked to said DNA molecule(s) and/or fragment(s).

Furthermore, the invention also pertains to constructs in form of "antisense" constructs comprising an "antisense" DNA with respect to the DNA molecules and fragments of the present invention.

The invention further relates to vectors, plasmids, cosmids, viral and phage genomes comprising one or more of the DNA molecules and fragments according to the present invention.

The invention also pertains to DNA molecules comprising a complementary sequence to the DNA molecules, fragments and constructs according to the present invention as well as the RNA transcription products of these DNA molecules, fragments and constructs.

For the isolation, construction, synthesis and modification of the DNA molecules, fragments and constructs according to the present invention various methods and techniques are known to the skilled scientist and are, e.g., described in Sambrook et al. (1989).

Furthermore, the invention pertains to polypeptides and proteins encoded by the DNA molecules, fragments and constructs according to the present invention, and in particular to a protein having or comprising the amino acid sequence as given in Figure 3 and SEQ. ID. NO. 1 and 2, proteins having or comprising an amino acid sequence exhibiting a homology of at least 65%, typically at least 75%, preferably at least 80%, more preferably at least 85%, even more preferably at least 90% and particularly preferred at least 95% to the amino acid sequence as given in Figure 3 and SEQ. ID. NO. 1 and 2, as well as polypeptide fragments of said proteins with the proviso that the amino acid sequences of those fragments exhibit a homology of at least 65%, typically at least 75%, preferably at least 80%, more preferably at least 85%, even more preferably at least 90% and particularly preferred at least 95% to a corresponding "reference" fragment of the polypeptide shown in Figure 3 and SEQ. ID. NO. 1 and 2.

In the present context, the term "polypeptide" includes molecules which typically comprise at least 18 amino acids, more particularly at least 25 amino acids and particularly preferred more than 40 amino acids.

In a particular embodiment of the invention, the polypeptides and proteins are fusion proteins of the proteins having acid phosphatase activity according to the invention, with an indicator protein or polypeptide as already outlined above. In a particular embodiment, a fusion protein according to the present invention comprises as said protein having acid phosphatase activity a protein having or comprising the amino acid sequence shown in Figure 3 and in SEQ. ID. NO. 1 and 2, or a protein homologous thereto as defined above.

In another embodiment, polypeptides and proteins are provided which comprise the polypeptides and proteins described in the last two paragraphs.

These polypeptides and proteins may be obtained by cultivating a host organism transformed with one or more of said DNA molecules and optionally purifying these polypeptides and proteins by methods known to the art. Alternatively, these polypeptides and proteins may i.a. also be prepared synthetically by use of known peptide and protein synthesizing techniques.

The polypeptides and proteins of the invention may be utilized for the preparation of monoclonal or polyclonal antibodies or, if they exhibit enzymatic activity, i.e. acid phosphatase activity, to utilize said enzymatic activity for various purposes.

Under a further aspect, the invention also covers monoclonal or polyclonal antibodies or antisera which specifically recognize and bind a polypeptide or protein according to the present invention. Corresponding antibodies may be prepared by use of the polypeptides or proteins according to the present invention for immunization of a host according to techniques known to the skilled scientist.

Furthermore, the invention includes transformed microorganisms, such as bacteria, bacteriophages, viruses, eucaryotic organisms like fungi, yeasts, protozoae, algae and human, animal and plant cells, which comprise a recombinant DNA sequence comprising at least one of the DNA molecules, fragments or constructs of the present invention. These transformed microorganisms may, i.a., be used as an expression system for producing the gene product(s) of the DNA molecules, fragments or constructs of the present invention. Typical microorganisms which are used for this purpose are bacteria, like *E. coli,* or yeasts such as *Saccharomyces cerevisiae.* Other types of transformed microorganisms according to the present invention, such as agrobacteria, like *Agrobacterium tumefaciens,* may be used for the transformation of plants with the inventive DNA molecules, fragments or constructs, thus providing transgenic plants.

Methods for transformation of microorganism cells with the DNA molecules, fragments or constructs of the present invention are well known to the skilled scientist, including the construction of expression vectors comprising the DNA sequences of the invention under the control of a constitutive or inducible promoter. The promoter may also enable a tissue-specific expression of the encoded information in particular compartments of an organism, such as a plant.

Finally, the invention pertains to transgenic plants, plant parts or seeds comprising a recombinant DNA sequence which comprises one or more DNA molecule(s) according to the present invention. The terms "plants" and "plant parts" include all of a plant propagation material such as a plant protoplast, a plant cell, a plant tissue, callus, a developing plantlet, a plant leaf, an immature whole plant and a mature whole plant. The transgenic plants, plant parts or seed may contain the said recombinant DNA sequence integrated into the genome thereof or extrachromosomally located. Various techiques and methods for the cloning of appropriate genes or gene fragments, construction of appropriate transformation vectors and the introduction of the genetic information contained in the vector into plant cells are known to the skilled scientist and enable the production of transgenic plants, plant parts or seeds according to the present invention. The techniques for introduction of genetic information into plant cells comprise a direct DNA transfer (e.g. into protoplasts by means of electroporation or by application of a high molecular weight osmotic agent as well as by biolistic methods wherein DNA-coated particles are shot into plant tissue), as well as the use of natural host/vector systems (e.g. of agrobacteria or plant viruses). For extrachromosomal maintenance of the recombinant DNA sequences, various specific viruses like tobacco mosaic virus (TMV) or potato virus X in the genome of which said recombinant DNA sequences then being inserted, may be utilized.

In a very particular embodiment, when utilizing for transformation a recombinant DNA sequence comprising an "antisense" construct according to the present invention (i.e. a construct comprising at least one DNA molecule or fragment according to the present invention in "antisense" orientation), transgenic plants, plant parts or seeds may be obtained which produce only little or no protein/polypeptide gene product of the DNA molecules according to the present invention, e.g. the acid phosphatase enzyme encoded by the nucleotide sequence given above. This technique has been established under the name "homology-dependent gene silencing" (see, for example, Taylor CB, 1997; Faske et al., 1997).

Exemplary plants for integration of the said recombinant DNA sequences of the invention include dicotyledonous plants as well as monocotyledonous plants, particularly cereals and crops, such as potato or tobacco.

To illustrate the invention, specific examples are set forth below. These examples are merely illustrative and are not to be understood as limiting the scope and underlying principles of the invention in any way. Various modifications of the invention in addition to those shown and described herein will become apparent to those skilled in the art from the following examples and foregoing description. Such modifications are also intended to fall within the scope of the appended claims.

### EXAMPLES

### Methods

Suppression subtractive hybridization (SSH) has been applied for the identification of relevant subsets of differentially expressed genes (Diatchenko et al., 1996). The technique is based on equalization of abundance of target cDNAs included in the tester cDNA population, and efficient subtraction of common sequences in tester and driver cDNA population by hybridization. In a specific form of PCR, differentially expressed target cDNAs are amplified selectively whereas the amplification of nontarget cDNA is suppressed.

We isolated differentially expressed cDNA fragments from chemically induced barley plants ("tester") when subtracted from cDNAs of non-induced plants ("driver").

### Plants, pathogens, and inoculation

Plants, namely the barley (*Hordeum vulgare L.*) cultivar Ingrid and the Rorl-2 mutant A89, were grown in a growth chamber at 16°C, 60 % relative humidity and a photo period of 16 h (100 µE s⁻¹ m⁻²). Control inoculation for chemical induction was done with 10 conidia mm⁻² from *Erysiphe graminis* DC: Fr. f.sp. *hordei,* isolate K1 (Hinze et al., 1991) at the third day after chemical treatment.

### Plant activation

2,6-Dichloroisonicotinic acid (DCINA, CGA 41396, Ciba Geigy AG, Basel, Switzerland), formulated as 25% active ingredient with a wettable powder (WP) carrier (Métraux et al. 1991), was applied to 5-days-old barley seedlings as a soil drench. The compound was used with final concentration of 10 mg 1⁻¹ soil volume. The suspensions were prepared with sterile tap water.

### Isolation of total RNA

For isolation of poly(A)⁺RNA used for SSH and race experiments the extraction of total RNA was done according to DUDLER et al. (1991) from 5 g leaf material.

For northern analysis total RNA was isolated by using RNA clean (AGS) with some modifications to the manual provided. Leaf material was grinded in liquid nitrogen. After adding 1,7 ml RNA clean extraction buffer and 200 µl chloroform to an aliquot of 200 mg leafpowder the content was thoroughly mixed and shaken for 30 minutes. The samples were centrifuged at 20800 x g (4 °C, 30 minutes) and the aqueous phase was extracted again with an equal volume of chloroform. After centrifugation (20800 x g, 4 °C, 15 minutes) RNA was precipitated out of the upper, aqueous phase with an equal volume of isopropanol, the samples were stored on ice for 1 hour and subsequently centrifuged at 20800 x g (4 °C, 30 minutes). The supernatant was discarded and the pellet washed once with 70 % ethanol (-20 °C). After spinning, the supernatant was thoroughly removed, the pellets were dried at room temperature and RNA was solubilized in an appropriate volume of water.

### Isolation of polyadenylated RNA (poly(A)+RNA)

Total RNA of different time points (12, 24, 48 hours after chemical induction and control treatment, respectively) was pooled with equal amounts. Pooled RNA was subjected to digestion with DNase as follows. Aliquots of 750 µg RNA were made up to 250 µl by adding 12,5 µl 1 M Tris-HCl (pH 7,5), 50 µl 50 mM MgCl₂, 1 µl 25 mM EDTA (pH 7,5), 2,5 µl RNase Inhibitor (40 U/µl), 10 µl DNaseI (10 U/µl) and the appropriate volume of water. The reaction mixture was incubated at 37 °C for 30 minutes in an air incubator. To extract the RNA the volume was made up to 1250 µl with water. A equal volume of phenol (saturated with Tris-HCl (pH 8,0)) : chloroform (3:1) was added, thoroughly mixed, and centrifuged at 20800 x g for 5 minutes at room temperature. The aqueous phase was transferred to a fresh tube and subsequently 0,1 volumes of 3 M sodium acetate (pH 5,2) and 2,5 volumes of cold (-20 °C) 96 % ethanol were added. The assays were mixed and stored at -20 °C for 24 hours. After centrifugation at 20800 x g for 1 hour at 4 °C the supernatant was discarded and the pellet washed with cold (-20 °C) 70 % ethanol. The mixtures were centrifuged again at 20800 x g for 15 minutes at 4 °C and the supernatant was removed. The pellets were dried at room temperature and dissolved in an appropriate volume of water.

Polyadenylated RNA (poly(A)⁺RNA) was isolated using the DYNABEADS mRNA Purification Kit (Dynal) according to the manufacturer's recommendations. The quality of the poly(A)⁺RNA preparation was checked in a denaturing 6 % polyacrylamid gel and silver staining as follows. For casting and running the gels a Mini-PROTEAN II cell (BioRad) was used. To 12 ml of the gel solution 5,6 µl Temed (SERVA) and 72 µl 10 % APS (BioRad) were added, the gel was poured immediately and allowed to polymerize for 2 hours. After a prerun at 100 V for 45 minutes, 100 ng of poly(A)⁺RNA and 250 ng of total RNA were denatured for 5 minutes in 5 x PAA-buffer at 98 °C, rapidly chilled on ice and loaded onto the gel. The run was performed at 60 V in 1 x TBE. After electrophoresis the gel was incubated 3 times for 15 minutes in fixing solution and rinsed with water 4 x 2 minutes and with 1,5 % (v/v) glycerol 1 x 2 minutes. 7 ml of staining solution A were mixed with 13 ml of staining solution B. The gel was submerged in this mix until bands became visible. The reaction was stopped by transferring the gel to the stop solution.

### cDNA Synthesis and generation of a subtracted library by suppression subtractive hybridization (SSH)

SSH was performed between A89 non-induced ("driver") and A89 chemically induced ("tester") using the PCR-Select™ cDNA Subtraction Kit (Clontech).

All procedures as first and second strand cDNA synthesis, *Rsa*I digestion and adaptor ligation were done according to the manufacturer's guidelines except modifications concerning the hybridization steps. For the first hybridization the mixtures of one non-ligated "driver" and one of the two different adaptor ligated "tester" cDNA-populations were denatured and kept at 68 °C for exactly 8 hours. For the second hybridization step the "driver-tester" hybridization assays were mixed, fresh denatured "driver" added and incubated at 68 °C for 12 hours. The following PCR reactions, done with the Advantage® cDNA Polymerase Mix (Clontech), were performed according to the manual provided with the subtraction kit. All PCR and hybridization steps were done in a Perkin-Elmer 2400 thermal cycler.

The PCR mixture now was enriched for differentially expressed cDNAs in roughly equal abundance.

### Cloning of the subtracted cDNAs into a TA vector

Although Advantage™ Polymerase Mixes only exhibit a minor amount of proofreading activity A-tailing was performed directly after the second PCR amplification to ensure that most of the cDNA fragments contain "A-overhangs". 10 µl of the reaction mix were incubated with dATP (final concentration of 0,4 mM) and 1 U Taq Polymerase (Eurogentec) for 15 minutes at 70 °C.

Without further purification 1 and 3 µl aliquots of the subtracted cDNA were ligated into 50 ng of pT-Adv (AdvanTAge™ PCR Cloning Kit, Clontech). After the ligation mixture was introduced into *E. coli* TOP10 F' (Clontech) the library was plated onto agar plates containing 100 µg/ml ampicillin, 50 µg/ml X-Gal and 280 µM IPTG. Bacteria were grown until colonies were visible and blue/white staining could be clearly distinguished. Plates then were stored at 4 °C.

### Colony PCR, reverse northern blots and screening

For evaluation of subtraction and the screening of true differentially expressed cDNAs, colony PCR was performed, the PCR-products blotted onto membranes and hybridized with Digoxygenin-labeled first strand cDNA deriving from pooled poly(A)⁺RNA extracted from induced and non-induced plants as template.

A total of 1200 white or light blue individual clones were picked and used to inoculate firstly agar plates containing 100 µg/ml ampicillin and secondly 96-well microtitre plates containing 40 µl sterile water. The bacteria in the microtitre plates were lysed by heating to 98 °C for 5 minutes using a Perkin-Elmer 9700 thermal cycler. PCR reaction mix was pipetted in 96-well microtitre plates as follows. Aliquots of 10 µl lysate were used to amplify cloned inserts in 30 µl reactions using a standard 10 x PCR buffer (Eurogentec) provided with the enzyme, 200 µM dNTPs, 1,5 mM MgCl₂, 0,5 U *Taq* polymerase (Eurogentec) and 0,4 µM of each primer (Nested 1 and Nested 2R) which flank the inserts. PCR was performed under the following conditions: one cycle of 94 °C for 3 minutes, 35 cycles each of 94 °C for 15 seconds, 68 °C for 30 seconds and 72 °C for 60 seconds, followed by one cycle of 72 °C for 5 minutes.

After amplification a pregel was run and a number of 360 clones were selected for reverse northern considering a minimum insert size of 0,2 kb. Two 14 µl aliquots were loaded onto a 1,5 % agarose/EtBr gel in 1 x TBE in parallel (gel A and gel B). Equal loading was monitored by fluorescence under UV-light due to EtBr accumulation by nucleic acids. The gels were blotted onto nylon membranes (Boehringer) in 2 x SSC for 16 hours. Filters A and B then were denatured for 2 x 5 minutes on whatman paper soaked with 0,5 M NaOH, 1,5 M NaCl and neutralized for 2 x 5 minutes on whatman paper soaked with 0,5 M Tris/HCl (pH 7,4), 1,5 M NaCl. Subsequently DNA was fixed by cross linking (GS Gene Linker, BioRad) with 125 mJ.

For first strand cDNA synthesis aliquots of 2 µg poly(A)⁺RNA with 2,22 µg oligo(dT₁₅) primer in a volume of 13 µl were heated to 65 °C for 10 minutes und chilled on ice for 5 minutes. 5 x first strand buffer (Boehringer), 7,5 µl dNTPs stock (2 mM dATP, dGTP, dCTP, 0,52 mM dTTP, 0,28 mM Dig-dUTP (Boehringer)) and 37,5 U RNase inhibitor (Boehringer) were made up to 15 µl with water and added to the poly(A)⁺RNA/primer mix. After incubation at room temperature for 2 minutes reverse transcription was started by adding 40 U MMuLV reverse transcriptase (Boehringer). The components were mixed thoroughly and incubated at 37 °C for 1 hour. The reaction was stopped by heating at 95 °C for 5 minutes and subsequently chilled on ice for another 5 minutes. For digestion of RNA RNaseA and RNaseH, 1 U of each was added to the first strand synthesis followed by an incubation at 37 °C for 1,5 hours.

### Control of Dig-labeled first strand cDNA

For screening of differentially expressed cDNAs using the reverse northern technique it is extremely important to estimate and equalize the intensity of Dig-incorporation into the populations of induced ("tester") and non-induced ("driver") single strand cDNAs.

Aliquots of first strand synthesis were denatured in an appropriate volume of RNA-loading buffer for 5 minutes at 95 °C, chilled on ice and loaded onto a denaturing 1,5 % agarose gel in 1 x MOPS and 5 % (v/v) Formaldehyd (37 %). After electrophoresis the probes were blotted onto a nylon membrane in 0,25 mM NaPO₄-buffer (pH 6,5) for 16 hours and then cross linked with 125 mJ (GS Gene Linker, BioRad). The Dig-labeled cDNAs were detected by chemiluminescence according to "The DIG System User's Guide for Filter Hybridization" (Boehringer). Concentration of Dig-labeled "tester" and "driver" probes was estimated by comparing signal intensities.

Screening for differentially expressed cDNAs was performed as follows. The filters (A and B) were hybridized in Dig Easy Hyb (Boehringer) for 16 hours at 68 °C with Dig-labeled single strand cDNAs derived from non-induced ("driver") and chemically induced ("tester") poly(A)⁺RNA, respectively. Filters were washed two times with 2 x SSC/0,1 % SDS at room temperature and another two times with 0,1 x SSC/0,1 % SDS at 50 °C. Detection by chemiluminescence was performed according to "The DIG System User's Guide for Filter Hybridization" (Boehringer).

### Isolation of plasmids

Bacteria were grown according to Sambrook et al. (1989) and plasmids isolated using the JETSTAR Plasmid Miniprep Kit (Genomed).

### Sequencing

Sequencing was performed using the Thermo Sequenase labeled primer cycle sequencing kit (Amersham) and Long Ranger Gel Solution (FMC) on a Licor 4200 sequencer (MWG-BIOTECH). The reactions were set up as follows. 2 pmol primer (5' labeled with IRD-800), 1,0 µg template DNA, 10 % (v/v) DMSO were made up to 21 µl with water. To 4,5 µl primer/template mix 1,5 µl of A, C, G or T reagent was added. Cycling was performed in a Perkin-Elmer 2400 thermal cycler with parameters as follows: one cycle of 95 °C for 5 minutes, 30 cycles each of 95 °C for 15 seconds, 57 °C for 15 seconds and 70 °C for 15 seconds, followed by one cycle of 70 °C for 5 minutes. The reaction was stopped by adding 5 µl stop buffer provided with the kit (Amersham). After a prerun of 30 minutes aliquots of 1 µl were loaded onto the gel. The run was performed as recommended by MWG-guideline to the sequencer.

### Sequence analysis

Homology searches in GenBank and EMBL databases were done using the BLAST algorithm (Altschul et al., 1990). Predicted amino acid sequences were aligned by using the CLUSTAL W program (Thompson et al., 1994).

The sequence analysis revealed similarity of the acid phosphatase enzyme from *Hordeum vulgare L.* to plants acid phosphatases and vegetative storage proteins, respectively. Similarity was highest to acid phosphatase in *Glycine max.* suggesting a functional relationship between the two proteins.

### Rapid Amplification of cDNA Ends (RACE)

5' and 3' ends of the cDNA were obtained by RACE using the MARATHON cDNA amplification kit (Clontech). For generating double-stranded cDNA, pooled poly(A)+RNA isolated from induced and non-induced plants, respectively (see above), served as template. After ligation of adaptors the library served as template for the amplification of 5' and 3' ends using gene specific primers in combination with adaptor primers provided with the kit. For obtaining the putative coding region of the Acid Phosphatase, 5'-race was performed with oligonucleotide 006GSP1 and 3'-race with oligonucleotide 006GSP2, both in combination with adaptor primer AP1. Race products were cloned into pT-Adv, propagated in *E. coli* TOP10F' and submitted to sequence analysis as described above.

### Northern blot analysis

To confirm differential expression northern blot analysis was performed by separating 5 to 10 µg total RNA on 1,5 % formaldehyde agarose gels (see above) and transferring to nylon membranes (Boehringer). Membranes were hybridized under stringent conditions in Dig Easy Hyb at 60 °C with Dig-PCR-labeled cDNA-probes deriving from SSH or race. Washing was carried out two times in 2 x SSC/0,1 % SDS at room temperature, then three times in 0,1 x SSC/0,1 % SDS at 60 °C. Detection was performed according to "The DIG System User's Guide for Filter Hybridization" (Boehringer).

### Material

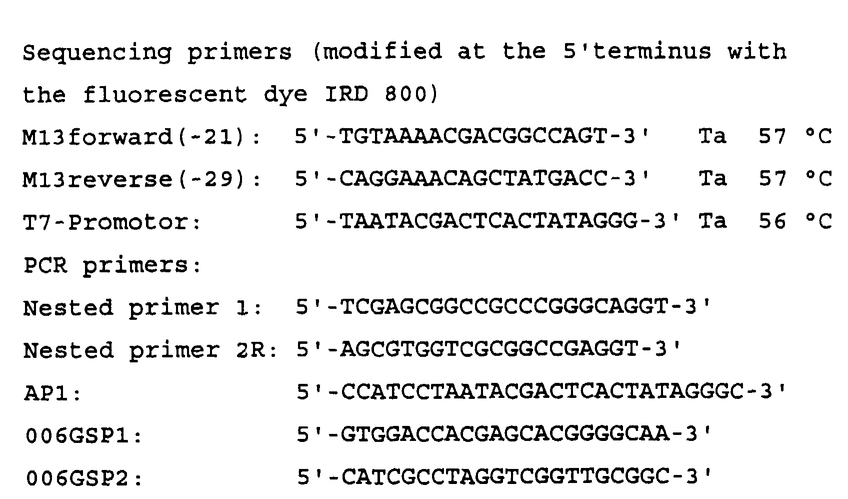

| Composition of the 6 % sequencing gel: | |
|---|---|
| 10 x TBE | 5 ml |
| Long Ranger Gel Solution (50 %) | 5 ml |
| Urea | 21 g |
| Water | ad 50 ml |
| TEMED | 25 µl |
| 10 % APS | 250 µl |

| 2 x SSC: | |
|---|---|
| NaCl | 0,3 M |
| Trisodium citrate | 30 mM |
| pH 7,0 | |

| RNA-loading buffer: | |
|---|---|
| Formaldehyde (37 %) | 260 µl |
| Formamide | 720 µl |
| Glycerol | 80 µl |
| Bromophenol blue (saturated solution in water) | 80 µl |
| 10 x MOPS | 180 µl |
| EtBr (10 mg/ml) | 100 µl |
| Water (Milli Q) | 100 µl |

| Fixing solution: | |
|---|---|
| Methanol | 45 % (v/v) |
| Acetic acid | 10 % (v/v) |
| in water | |

| Staining solution A: | |
|---|---|
| Na₂CO₃ | 5 % (w/v) in water |

| Staining solution B: | |
|---|---|
| NH₄NO₃ | 0,2 % (w/v) |
| AgNO₃ | 0,2 % (w/v) |
| Tungstosilicic acid | 1 % (w/v) |
| Formaldehyde | 1,4 % (v/v) |
| in water | |

| Stop solution: | |
|---|---|
| Acetic acid | 10 % (v/v) |
| Glycerol | 10 % (v/v) |

| 5 x PAA-buffer: | |
|---|---|
| Urea | 7 M |
| Bromophenol blue | 0,25 % (w/v) |
| Xylene cyanol | 0,25 % (w/v) |
| EDTA | 10 mM |
| Glycerol | 30 % (v/v) |

| Gel solution: | |
|---|---|
| Urea | 7 M |
| Acrylamide solution (37,5:1 acrylamide:bisacrylamide) | 11,625 ml |
| 10 x TBE | 7,5 ml |
| Water | ad 75 ml |

| 10 x TBE: | |
|---|---|
| Tris base | 0,9 M |
| Boric acid | 0,9 M |
| EDTA | 25 mM |

### REFERENCES:

Altschul, Stephen F., Thomas L. Madden, Alejandro A. Schäffer, Jinghui Zhang, Zheng Zhang, Webb Miller, and David J. Lipman (1997) Gapped BLAST and PSI-BLAST: a new generation of protein database search programs. Nucleic Acids Res. 25, 3389-3402.

Diatchenko, L., Lau; Y.-F., Campbell, A. P., Chenchik, A., Moqadam, B.H., Lukyanov, S., Lukyanov, K., Gurskaya, N., Sverdlov, E.D. and Siebert, P.D. (1996) Proc. Natl. Acad. Sci. USA 93, 6025-6030.

Dudler, R., Hertig, C., Rebmann, G., Bull, J., and Mauch, F. (1991) A Pathogen-Induced Wheat Gene Encodes a Protein Homologous to Glutatione-S-Transferases. MPMI 4, 14-18.

Faske, M., Backhausen, J.E., Sendker, M., Singer-Bayrle, M., Scheibe, R., von Schaewen, A. (1997) Transgenic tobacco plants expressing pea chloroplast *Nmdh* cDNA in sense and antisense orientation: Effects on NADP-MDH level, stability of transformants, and plant growth. Plant Physiol. 115, 705-715.

Gottlieb, M. and Chavko, M. (1987) Silver staining of native and denatured eucaryotic DNA in agarose gels. Analyt. Biochem. 165, 33-37.

Hinze, K., Thompson, R.D., Ritter, E., Salamini, F., and Schulze-Lefert, P. (1991). RFLP-mediated targeting of the mlo resistance locus in barley (Hordeum vulgare). Proc. Natl. Acad. Sci. USA 88, 3691-3695.

Mason, H.S, and Mullet, J.E. (1990). Expression of two soybean vegetative storage protein genes during development and in response to water deficit, wounding and jasmonic acid. Plant Cell 2, 569-579

Mason, H.S., DeWald, D.B., and Mullet, J.E. (1993). Identification of a methyl jasmonate-responsive domaine in the soybean vspB promoter. Plant Cell 5, 241-251

Berger, S., Bell, E., Sadka, A., and Mullet, J.E. (1995). Arabidopsis thaliana Atvsp is homologous to soybean VspA and VspB genes encoding vegetative storage protein acid phosphatases, and is regulated similarly by methyl jasmonate, wounding, sugars, light and phosphate. Plant Mol. Biol. 1995, 2, 933-942

Métraux, J.P., Ahl-Goy, P., Staub, T., Speich, T., Steinemann, A., Ryals, J., and Ward, E. (1991) Induced resistance in cucumber in response to 2,6- dichloroisonicotinic acid and pathogens. In Henneke, H., und Verma, D.P.S., eds., Advances in Molecular Genetics of Plant-Microbe Interactions, Vol 1. Kluwer Academic Publishers, Dordrecht, The Netherlands, pp 432-439

Sambrook, J., Fritsch, E.F., and Maniatis, T. (1989) Molecular cloning: a laboratory manual, 2nd edn, Cold Spring Harbour Laboratory, Cold Spring Harbour NY.

Taylor, C.B. (1997) Comprehending cosuppression. Plant Cell 9, 1245-1249

Thompson J.D., Higgins D.G., Gibson T.J. (1994) CLUSTAL W: improving the sensitivity of progressive multiple sequence alignment through sequence weighting, position-specific gap penalties and weight matrix choice. Nucleic Acids Res. 22, 4673-4680.

## Claims

1. A method of testing a chemical compound for its capability to induce an enhanced resistance status (ERS) in plants said method comprising:
(a) contacting a plant or plant part comprising a gene fragment coding for an acid phosphatase the expression of which being inducible by SAR inducing compounds, with the chemical compound; and
(b) assaying the expression of said gene fragment,
wherein the expression of said gene fragment indicates that the chemical compound has the capability to induce ERS.

2. The method according to claim 1
wherein the expression of the gene fragment coding for an acid phosphatase is not inducible by plant pathogens.

3. The method according to claim 1 or 2
wherein the expression of the gene fragment coding for an acid phosphatase is further inducible by ISR inducing compounds.

4. The method according to any one of claims 1 to 3 wherein the said gene fragment coding for an acid phosphatase is a gene fragment comprising the following nucleotide sequence:

ATGCTCGGCAACCACTTCCGGCGCGACTGCAAGGTCGTCATCGACCAGGCCATCGCCTACGTCG
ACGGCCTCAAGCTCGCCGGCAACGGCAAGGACGTGTGGGTCTTCGACATCGACGAGACCACCCT
CTCCAACCTCCCTTACTACGCCACGCACGGCTTTGGGGCTAGGCCATACAACGCGACGAGCTTC
GACGCGTACGTGATGGAGGGGAGTGCACCGGTGCTGCCGGAGACGAAGCGGCTCTACTACAAGC
TACTCAAGGTGGGCATCAAGCCGGTATTCATCACAGGCCGGACGGAGGATAAGAGGGCCGTCAC
CGTCGGGAACCTCCGCAGCCAGGGCTTCTCCGGGTGGATGAGCCTGACGCTCAAGCAACATGGG
TTCAAGGGCTCCGCCATATCCTACAAGTCCGCCGAGAGGAAGAAGCTGGAGGATGCCGGGTACG
TCATCGTCGGCAACATCGGCGACCAGTGGAGCGACATCCTCGGCGCGCCCGAGGGCGCTCGCAC
CTTCAGGCTGCCTGACCCCATGTACTACATCGCCTAG.

5. The method according to any one of claims 1 to 3 wherein the said gene fragment coding for an acid phosphatase is a gene fragment having substantial homology with the nucleotide sequence given in claim 4, a variant of the gene fragment defined in claim 4 or a gene fragment comprising one or more portions of the aforementioned gene fragments and variants.

6. The method according to any one of claims 1 to 5 wherein the said plant or plant part is selected from the group consisting of a plant protoplast, a plant cell, a plant tissue, callus, a developing plantlet, an immature whole plant, a mature whole plant and seed.

7. The method according to any one of claims 1 to 6 wherein the said plant or plant part is a cereal, and in particular a plant of the genus hordeum, or a plant part derived therefrom.

8. The method according to any one of claims 1 to 7 wherein the gene fragment coding for an acid phosphatase further comprises a nucleotide sequence coding for an indicator protein or polypeptide and wherein the assaying of the expression of the said gene fragment is carried out by monitoring the expression of the nucleotide sequence coding for the indicator protein or polypeptide.

9. A method for screening for chemical compounds, in particular agrochemicals, having the capability to induce ERS in plants wherein the method according to any one of claims 1 to 8 is used and wherein the chemical compounds to be screened are provided in form of a synthetic combinatorial library of chemical compounds and/or in form of a library of natural products.

10. Kit for use in the methods according to any one of claims 1 to 9 which comprises the following components:
(a) one or more plant or plant parts comprising a gene fragment coding for an acid phosphatase the expression of which being inducible by SAR inducing compounds and optionally also by ISR inducing compounds; and
(b) means for detecting the expression of the said gene fragment.

11. A process for providing chemical compounds having the capability of inducing ERS in plants which comprises the steps of
(a) generating a synthetic combinatorial library of chemical compounds, and
(b) screening the compounds of said library with a method as claimed in claim 9.

12. A new compound having the capability of inducing ERS obtainable by a process according to claim 11.

13. Use of a compound according to claim 12 as agrochemical compound, and in particular for the induction of ERS in plants.

14. An isolated DNA molecule which codes for a protein or polypeptide having the biological activity of the enzyme acid phosphatase the expression of which being inducible by SAR inducing compounds if said DNA molecule is situated within its natural genetic environment, or a nucleotide sequence complementary thereto.

15. An isolated DNA molecule according to claim 14 which comprises the following nucleotide sequence: 
ATGCTCGGCAACCACTTCCGGCGCGACTGCAAGGTCGTCATCGACCAGGCCATCGCCTACGTCG
ACGGCCTCAAGCTCGCCGGCAACGGCAAGGACGTGTGGGTCTTCGACATCGACGAGACCACCCT
CTCCAACCTCCCTTACTACGCCACGCACGGCTTTGGGGCTAGGCCATACAACGCGACGAGCTTC
GACGCGTACGTGATGGAGGGGAGTGCACCGGTGCTGCCGGAGACGAAGCGGCTCTACTACAAGC
TACTCAAGGTGGGCATCAAGCCGGTATTCATCACAGGCCGGACGGAGGATAAGAGGGCCGTCAC
 
CGTCGGGAACCTCCGCAGCCAGGGCTTCTCCGGGTGGATGAGCCTGACGCTCAAGCAACATGGG
TTCAAGGGCTCCGCCATATCCTACAAGTCCGCCGAGAGGAAGAAGCTGGAGGATGCCGGGTACG
TCATCGTCGGCAACATCGGCGACCAGTGGAGCGACATCCTCGGCGCGCCCGAGGGCGCTCGCAC
CTTCAGGCTGCCTGACCCCATGTACTACATCGCCTAG
 or a nucleotide sequence complementary thereto.

16. An isolated DNA molecule, the nucleotide sequence of which having substantial homology with the nucleotide sequence as given in claim 15, in particular hybridizes with the latter, or is a variant of the DNA molecule of claim 15 or is related to the DNA molecule of claim 15 by mutation.

17. A DNA molecule encoding the acid phosphatase enzyme having the amino acid sequence given in SEQ ID NO. 2.

18. Vector, plasmid, cosmid, viral or phage genome comprising at least one of the DNA molecules as defined in any of claims 14 to 17.

19. A polypeptide or protein encoded by any of the DNA molecules as defined in any one of claims 14 to 17.

20. A monoclonal or polyclonal antibody which specifically recognizes and binds a polypeptide or protein as defined in claim 19.

21. A transgenic plant, plant part or seed comprising a recombinant DNA sequence which comprises the DNA molecule according to any one of claims 14 to 17.
